# EUROPEAN PATENT APPLICATION

(11) **EP 4 541 360 A1**
(43) Date of publication of application: **23.04.2025**
(21) Application number: 22946069.6
(22) Date of filing: 29.07.2022
(51) Int. Cl.: A61K 35/742, A61K 35/747, A61K 36/06, A23K 10/18, A23K 50/70, A61P 43/00

(54) **LIQUID PROBIOTIC COMPOSITION FOR GREATER TOLERANCE TO HEAT STRESS IN POULTRY, METHOD OF PRODUCING THE COMPOSITION AND USE THEREOF IN THE TREATMENT OF POULTRY**

(30) Priority: 15.06.2022 BR 102022011914
(71) Applicant: Total Biotecnologia Indùstria e Comêrcio S/A, 81460-020 Curitiba (PR) (BR); Biovirtus Soluções Ambientais Ltda, 13289-014 Vinhedo (SP) (BR)
(72) Inventor: SANDANIEL ZEM, Tânia Mara, 13289-014 Vinhedo (SP) (BR); DOUGLAS FABIANO, Gomes, 13289-014 Vinhedo (SP) (BR)
(74) Representative: Boco IP Oy Ab
(86) International application number: PCT/BR2022/050298
(87) International publication number: WO 2023/240326

(57) **Abstract**

The present invention is related to the industrial process and use of a liquid probiotic composition administered to poultry for higher tolerance to heat stress, meaning elevated temperatures. The technical solution relative to the present invention employs a microbiological consortium of four bacteria; Rhodopseudomonas palustris, Lactobacillus plantarum, Bacillus amyloliquefaciens, B. licheniformis, as well as a yeast-like fungus; Saccharomyces cerevisiae. In a surprising manner, when applied from the initial stages in poultry, the present invention presents preventive reduction of the deleterious effects caused by high temperatures, resulting in greater zootechnical performance viability of the animals without the need for applying antimicrobial food additives and growth promoters.

## Description

### FIELD OF THE INVENTION (TECHNICAL SECTOR)

The present invention is related to an industrial process for obtaining a probiotic composition with one or more species of the genera *Rhodopseudomonas, Lactobacillus, Bacillus* and *Saccharomyces,* as well as the application thereof through drinking water, with the purpose of reducing mortality in poultry in face of heat stress (high temperature), as well as additional benefits relative to the various zootechnical indexes that are important for poultry farming without the need for applying antimicrobial food additives and growth promoters.

### DESCRIPTION OF THE RELATED TECHNIQUE

Brazilian poultry farming presents great worldwide relevance, since the country is the largest chicken meat producer and exporter. According to a USDA (United States Department of Agriculture) report, Brazil should continue as largest exporter of this animal protein, reaching 5,2 million tons by 2031 (https://apps.fas.usda.gov/psdonline/circulars/livestock poultry.pdf).

To support the projected growth, poultry farming resorts to different strategies, so as to achieve greater productivity and overcome the challenges faced by the sector. Among the most important factors which impact negatively world poultry farming are the sanitary conditions, the environmental conditions and the high production cost.

As regards health, the health of the animal directly reflects on the poultry creation yield, since controlling pathogens reduces the mortality of the poultry, contributing to a better zootechnical performance. The infections caused by *Salmonella* spp., called salmonellosis, are among the most relevant ones. There are more than 2.600 serotypes of bacteria of the genus *Salmonella* identified as potential disseminators of zoonotic infections in humans **[**Tessari, E. N. C., Kanashiro, A. M. I., Stoppa, G. F., Luciano, R. L., Castro, A. G. M. & Cardoso, A. L. S. P. Important aspects of Salmonella in the poultry industry and in public health. In Mahmoud, B.S.M. (Ed.), Salmonella-A dangerous foodborne pathogen. Croatia: IntechOpen, 2012**],** which demands a great effort in controlling this pathogen, by means of strict measures, which, in Brazil, are regulated by the Ministry of Agriculture, Livestock and Supply (MAPA) , by means of the National Avian Health Surveillance Program (PNSA) **[**Raposo, R.S., Defensor, R.H., Grahl, T.R. Use of probiotics in poultry farming for control of Salmonella spp.: literature review and utilization perspectives. Pubvet, v. 13 No. 04 p. 152, 2019**].**

It is worth mentioning that the conventional method for controlling zoonoses, such as the infections triggered by *Salmonella* employs the use of antimicrobial compounds **[**Gut, A. M., Vasiljevic, T., Yeager, T. & Donkor, O. Salmonella infection-prevention and treatment by antibiotics and probiotic yeasts: a review. Microbiology, 164(11):1327-1344.), 2018**].** However, the extensive use of these compounds has promoted the selection of resistant infectious agents and highly virulent, resulting in the prohibition of this practice by the European Union. And, as a reflex of this prohibition, there was great difficulty in maintaining the poultry activity levels without the possibility of applying growth promoting antimicrobial food additives, creating room for the adoption of probiotic products, a sustainable approach which has shown promising results to the sector **[**Patterson, J.A., Burkholder, K.M. Application of prebiotics and probiotics in poultry production. Poultry Science, v.82, n.4, p.627-631, 2003**;** Revington, B. Feeding poultry in the post-antibiotic era. In: MULTI-STATE POULTRY MEETING, Indiana, U.S.A. Accessed on 09 may, 2022. Online. Available at http://ag.ansc.purdue.edu/poultry/multistate/Multistate.pdf ].

As relevant as health, the environmental conditions can strongly interfere with the results reached by industrial poultry farming. In this sense, high investments are dedicated to minimizing climatic conditions, whereby the most important are relative to the temperature. Depending on the development phase of the animal, the optimum temperature conditions also vary. Thus, investments in equipment destined to maintain thermal comfort are extremely relevant for the success of the avian activity, however, they reflect directly on the production costs.

Among the different embodiments of abiotic stresses, that caused by exposure to high temperatures is among the most impacting for avian production. Excessive heat is responsible for strong impacts on the physiological balance and homeostasis of the animals, reflecting in negative effects on the performance and health of the poultry **[**El-Hack, M.E., El-Saadony, M.T., Shafi, M.E., Qattan, S.Y.A., Batiha, G.E., Khafaga, A.F. Abdel-Moneim, Abdel-Moneim, E. Mahmoud Alagawany, A.M.M. Probiotics in poultry feed: A comprehensive review. J. Anim. Physiol. Anim. Nutr. 104:1835□01850, 2020**].** During heat stress periods, multiple body temperature control mechanisms are activated. However, when the stress to which the animal is submitted covers long periods, it is common to observe an increase in mortality due to immune system disorders, endocrine imbalances and osmotic imbalances **[**Khafaga, A. F., Noreldin, A. E., Taha, A. E. The adaptogenic anti-ageing potential of resveratrol against heat stress-mediated liver injury in aged rats: Role of HSP70 and NF-kB signalling. Journal of Thermal Biology, 83, 8D21, 2019**;** Sohail, M. U., Hume, M. E., Byrd, J. A., Nisbet, D. J., Ijaz, A., Sohail, A., Shabbir, M. Z., Rehman, H. Effect of supplementation of prebiotic mannanoligosaccharides and probiotic mixture on growth performance of broilers subjected to chronic heat stress. Poultry Science, 91, 2235D2240, 2012**].** Finally, the heat stress can impact the gastrointestinal microbiome of the animals, including the population of microorganisms which assist in promoting animal health **[**Sohail, M.U., Ijaz, A., Younus, M. , Shabbir, M.Z., Kamran, Z. , Ahmad, S., Anwar, H., Yousaf M.S., Ashraf K., Shahzad A.H., Rehman H. Effect of supplementation of mannanoligosaccharide and probiotic on growth performance, relative weights of viscera, and population of selected intestinal bacteria in cyclic heat-stressed broilers. J. Appl. Poult. Res., 22: 485D491, 2013**].** As a result, the decrease of beneficial microorganisms creates a prerogative for increasing pathogens, extending the risk to the consumption of animal meats which have gone through high temperature conditions **[**Traub-Dargatz, J. L., Ladely, S. R., Dargatz, D. A., Fedorka-Cray, P.F. Impact of heat stress on the fecal shedding patterns of Salmonella enterica typhimurium DT104 and Salmonella enterica infantisby 5-week-old male broilers. Foodborne Pathogens and Diseases,3, 1780183, 2006**].**

In the search for solutions to the negative effects caused by heat stress, countless studies have characterized the beneficial effect of probiotics applied to animals submitted to this stress modality. Sohail and collaborators (2012) and (2013), as well as Lei and collaborators (2013), demonstrated the beneficial effect of probiotics over the morphology of the intestinal tract of animals submitted to high temperatures **[**Lei, K. , Li, Y.L., Yu, D.Y., Rajput, I.R., Li, W.F. Influence of dietary inclusion of Bacillus licheniformis on laying performance, egg quality, antioxidant enzyme activities, and intestinal barrier function of laying hens. Poult. Sci., 92: 2389□2395, 2013**].**

Landy and Kavyani (2013) demonstrated that probiotics present potential for mitigating the commitment of the immune system of the animals submitted to heat stress **[**Landy, N., Kavyani, A. Effects of using a multi-strain probiotic on performance, immune responses and cecal microflora composition in broiler chickens reared under cyclic heat stress condition. Iran. J. Appl. Anim. Sci., 3: 703□708, 2013**].** However, Sohail and collaborators (2012) did not evidence beneficial effects on the immunity of chickens submitted to heat stress treated with probiotics based on *Lactobacillus (L. plantarum, L. acidophilus, L. bulgaricus, L. rhamnosus, B. bifidum, S. thermophilusr E. faecium, A. oryzae* and C. *pintolopesii).* Similar results, that is, without positive effect on the immunity, were found by Rahimi and Khaksefidi (2006), when they tested a probiotic product composed of *Bacillus subtilis* and *B. licheniformis* **[**Rahimi, S.H., Khaksefidi, A. A comparison between the effects of a probiotic (Bioplus 2B) and an antibiotic (virginiamycin) on the performance of broiler chickens under heat stress condition. Iran. J. Vet. Res., 7: 23□28, 2006**].**

AL-fataftah and Abdelgader (2014) demonstrated that food supplementation in chickens submitted to heat stress with *B. subtilis* proved effective for the growth of the animals **[**AL-Fataftah, A.R., Abdelqader, A. Effects of dietary Bacillus subtilis on heat-stressed broilers performance, intestinal morphology and microflora composition. Anim. Feed Sci. Technol., 198: 279□285, 2014**].**

With similar results, Song and collaborators (2014) observed that supplementing the diet of chickens in high temperature conditions with *B. subtilis, B. licheniforms* and *L. plantarum,* resulted in better food conversion rate, obtained by the ratio between feed consumption and weight gain **[**Song, J., Xiao, K. , Ke, Y.L., Jiao, L.F., Hu, C.H., Diao, Q.Y., Shi, B., Zou, X.T. Effect of a probiotic mixture on intestinal microflora, morphology, and barrier integrity of broilers subjected to heat stress. Poult. Sci., 93: 581□588, 2014**].**

The benefit of using probiotic additives in poultry diets is recognized, independent of the condition to which the animals are submitted. However, Lara and Rostagno (2013) concluded that the beneficial effect of probiotics in poultry is not clearly understood and that it is supposedly related with the compositions and characteristics of the microorganisms used as probiotics **[**Lara, L.J., Rostagno, M.H. Impact of heat stress on poultry production. Animals, 3:356□369, 2013**].**

North American patent US6410016(2002) presents a method for administering viable microorganism compositions to poultry. The document describes the use of *Lactobacillus reuteri, L. johnsonii* and *Bacillus subtilis* in different phases of the lifecycle of the poultry.

US9247757 (2016) proposes a method for using *B. subtilis* for increasing the health of the animals. It is limited to strain QST 713 and mutants thereof.

US20070202088 (2007) discloses a new microorganism composition for treating and preventing diseases in poultry. The document states that administering one or more species of *Bacillus* can inhibit diseases caused by *Escherichia coli, Salmonella* and *Clostridium* in poultry.

Document WO2004104175 (20041) describes methods for identifying probiotic bacteria for poultry supplement. The assessments of the probiotic effects are made by analyses of the alterations in the intestinal microflora of the poultry resulting from probiotic supplementation.

The documents cited to bring to light the state of the art, among scientific works and patents, exemplify and evidence the importance of probiotics for poultry farming in what concerns the health and performance of the animals. However, the teachings present in the state of the art do not achieve a liquid probiotic composition really able to reduce mortality in poultry submitted to heat stress. And, neither do they increase the avian activity yield, by means of oral administration through water and food. Thus, the development of probiotic compositions with said characteristics is interesting.

### BRIEF DESCRIPTION OF THE INVENTION

Thus, the present invention is related to an original concept, of liquid nature, administered through drinking water, with the purpose of reducing mortality in poultry submitted to heat stress and increasing the avian activity yield.

The first embodiment of the invention is characterized by the use of a microbial consortium as probiotic food additive for poultry, constituting a composition which combines at least the following species of probiotics: *Rhodopseudomonas palustris, Bacillus licheniformisr B. amyloliquefaciens, Lactobacillus plantarum* and *Saccharomyces cerevisiae.*

More specifically, the microbial consortium object of the present invention is composed of *Rhodopseudomonas palustris* CCTB18, *Saccharomyces cerevisiae* CCTB12, *Bacillus amyloliquefaciens* CCTB06, *B. licheniformis* CCTB07 and *Lactobacillus plantarum* CCTB27.

In a second embodiment of the invention, we have the method for obtaining the probiotic which preferably consists of the batch fermentation cultivation, wherein each species is produced separately, employing specific cultivation means, as well as growth conditions defined by the present invention.

The constitution of the composition occurs by the mixture of different microorganisms in a process which allows the homogeneity of the microbiological consortium.

The composition obtained by the present invention presents liquid physical nature characterized as a concentrated suspension of viable microorganisms.

In a third embodiment of the invention, we have the use of a liquid probiotic composition in the manufacture of veterinary medications for administration, preferably through drinking water offered to the animals.

The present invention teaches the administration of a probiotic composition with liquid nature through drinking water, which, carried out from the initial life stages, increases the tolerance of the poultry in face of heat stress (high temperatures) excluding the need to apply antimicrobial food additives and growth promoters.

### DETAILED DESCRIPTION OF THE INVENTION

The microorganisms used in the present invention were isolated from soil samples collected in the district of Lapa, state of Paraná, Brazil. The geolocation of the collection meets at coordinates 25°50'49"S and 49°39' 09.67" W.

The processing of the samples for isolating the bacteria was carried out using specific culture medium which favors the growth of groups of microorganisms described in table 1.

**TABLE 1: CULTURE MEDIUM EMPLOYED IN ISOLATING MICROORGANISMS WHICH COMPOSE THE PRESENT INVENTION.**

| | ***Reagents*** | *Rhodopse udomonas* | *Saccharo myces* | *Lactobac illus* | *Bacillus* |
|---|---|---|---|---|---|
| **04** | Agar | 10 - 15 g | 10 - 15 g | 10 -15 g | 10 - 15g |
| **05** | Yeast extract | 5 - 10g | 5-15g | 1 - 5 g | - |
| **06** | Meat extracts | - | - | 0,2 - 4g | 1 - 5 g |
| **07** | Sucrose | - | - | 0,05 - 1mL | - |
| **08** | Peptone | - | - | 0,05 - 1mL | 5 - 10 g |
| **09** | Glycero l | - | - | 5 - 20mL | - |
| **10** | KNO3 | - | - | 0,5 - 3g | - |
| **11** | Malic acid | 1 - 5g | - | - | - |
| **12** | Sucrose | - | 5-15g | - | - |
| **13** | Peptone | - | 2-5g | - | - |
| **14** | Water | q.s.p. 1L | q.s.p. 1L | q.s.p. 1L | q.s.p. 1L |

| | | | | | |
|---|---|---|---|---|---|
| q.s.p. quantity sufficient for | | | | | |

From the isolated colonies there were sequenced preserved genes, 16 S for bacteria and 18 S for fungi, for determining the genus and species of the respective microorganisms.

After the taxonomic identification of the microorganisms which compose the probiotic, codes for identification of the isolated were attributed, which codes are named Total Biotecnologia Culture Collection (CCTB) (Coleção de Culturas Total Biotecnologia).

Thus, in the first embodiment of the present invention we have a liquid probiotic composition for use as a food additive for poultry through administration by the drinking water, being composed of four species of bacteria: 50.0010 to 99.995% of *Rhodopseudomonas palustris* CCTB18, 0.001% to 49.995% of *Lactobacillus plantarum* CCTB27, 0.0010 to 49.995% of *Bacillus amyloliquefaciens* CCTB06 and 0.001%. to 49.995% of *Bacillus licheniformis* CCTB07, as well as 0.001% to 49.995% of a species of a yeast-like fungus; 0.001% to 49.995% of *Saccharomyces cerevisiae* CCTB12. The composition can be complemented with excipients, veterinary carriers and other products suitable for oral treatment of poultry with heat stress.

In the second embodiment, the present invention provides a production process for a liquid probiotic composition in a method which comprises the steps of:

(a) isolated batch fermentation of the microorganisms, namely *Rhodopseudomonas palustris* CCTB18, *Lactobacillus plantarum* CCTB27, *Bacillus amyloliquefaciens* CCTB06, *Bacillus licheniformis* CCTB07 and *Saccharomyces cerevisiae* CCTB12 able to improve the zootechnical parameters of avian activity as a consequence of the higher tolerance to heat stress, by means of the specific formulation for each microorganism during the industrial process; and

(b) formulation of a biotechnological product composed by a mixture of bacteria and yeast-like fungus, in a technical solution which allows application in the avian culture through the drinking water offered to the animals as probiotic additive.

In a preferred embodiment, according to the present invention, the fermentation (step (a)) of the microorganisms of the genera *Rhodopseudomonas, Lactobacillus, Bacillusand Saccharomyces* takes place, preferably, by batch and occurs for approximately 8 - 168 hours.

In a preferred embodiment, the method of the present invention comprises the sequential expansion (scaling) of the cultures of *Rhodopseudomonas, Lactobacillus, Bacillus* and *Saccharomyces* for inoculation of the fermentation culture.

Preferably, the sequential expansion is started at 100 mL volumes, which serve as inoculum for 1 L. This, in turn, is inoculated in 10 L, which, then, are inoculated in 180 L tanks and which, finally, are transferred to reactors containing 2.000 L.

In a preferred embodiment, the species of *Rhodopseudomonas, Lactobacillus, Bacillus* and *Saccharomyces* are expanded in 100 mL flasks for incubation in orbital agitator of 80 rpm to 200 rpm. The incubation time is preferably 8 hours to 48 hours.

Preferably, the species of *Rhodopseudomonas, Lactobacillus and Saccharomyces* are cultivated in flasks with around 1 L of culture medium per incubation in orbital agitator between 80 rpm to 200 rpm. Preferably, the species of *Bacillus, B. licheniformis* and *B. amyloliquefaciens,* are then cultivated in stainless-steel fermentation units containing about 1 L of culture medium. The incubation time is preferably about 8 hours to about 48 hours with airflow of about 0,25 Nm³/h to about 1,0 Nm³/h (=4,16 - 16,67 vvm).

In a preferred embodiment, for all the species that make up the present invention, the airflow of the stainless-steel fermentation units containing about 10 L culture and about 0,25 to about 1,5 Nm³/h (= 0,41 - 2,5 vvm), and the incubation time is preferably about 8 hours to about 48 hours.

In a preferred embodiment, the incubation temperature for multiplication of the species of *Rhodopseudomonas, Lactobacillus, Bacillus* and *Saccharomyces* according to the present invention is 22 °C to 38 °C.

In a preferred embodiment, the species of *Rhodopseudomonas, Lactobacillus, Bacillus* and *Saccharomyces* are inoculated separately in the scaling process until 2.000 L culture and mixed in bioreactors or stainless-steel mixers, previously sterilized for about 30 minutes at 121 °C and 1,8 Kgf/cm², of 10.000 L as described for the present invention.

Preferably, the culture medium for the scales up to 10 L, 180 L and 2.000 1 are specific for each genus of microorganism. The airflow is, preferably, of 1,0 to 10,0 Nm³/h and the incubation occurs for 24 to 168 hours.

In a preferred embodiment, the mixing step of the species of *Rhodopseudomonas, Lactobacillus, Bacillus* and *Saccharomyces is* carried out at a temperature of 22 °C to 38 °C. The airflow is preferably of 1,0 Nm³/h to 10,0 Nm³/h. The pressure is preferably of 0,5 to 1,2 kgf/cm². The agitation is preferably of 40 hz to 45 hz.

The constitution of the product occurs by the mixture of the different microorganisms cultivated separately by batch until the step of 2.000 L in bioreactors or mixed with a volume of 10.000 L in a process which allows homogeneity of the microbiological consortium, accessed by constant agitation between 40 hz to 45 hz for 0,5 to 2 hours.

The product obtained by the present invention presents liquid physical nature characterized as a concentrated suspension of viable microorganisms.

In a preferred embodiment, the product of the mixture of one or more species of the genera *Rhodopseudomonas, Lactobacillus, Bacillus* and *Saccharomyces* are bottled in polypropylene flasks with volumes of 0,5 L, 1,0 L, 2,0 L, 5,0 L, 10,0 L and 20,0 L.

In a preferred embodiment, the method of administering the product described in the present invention occurs by means of the drinking water offered to the animals in doses between 0,1 L to 10,0 L for every 1.000 L of water.

In a preferred embodiment, the present invention teaches that the administration of the probiotic product with liquid nature through the drinking water from birth of the birds until slaughter, increases the tolerance of the animals to heat stress (high temperatures) between 30°C to 38 °C, reducing mortality and increasing zootechnical parameters.

### EXAMPLES

### EXAMPLE 1 - SCALING OF THE CULTURE

The different species of *Rhodopseudomonas, Lactobacillus, Saccharomyces* and *Bacillus* are inoculated separately from the initial step in 100 mL of culture medium until the last step of the scaling, 2.000 L culture, according to the compositions described in Tables 2, 3 and 4.

**TABLE 2. CULTURE MEDIUM USED FOR GROWTH OF RHODOPSEUDOMONAS, SACCHAROMYCES AND LACTOBACILLUS UP TO THE SCALE OF 2.000 L.**

| | ***Reagents*** | *Rhodopseudomo nas* | *Saccharomyc es* | *Lactobacil lus* |
|---|---|---|---|---|
| **01** | K₂HPO₄ | 0,1 - 2g | - | 0,1 - 1g |
| **02** | KH₂PO₄ | - | - | 0,1 - 1g |
| **03** | MgSO₄.7H₂O | 0,05- 1g | - | 0,1- 0,1g |
| **04** | NaCl | - | - | 0,05 - 0,3g |
| **05** | Yeast extract | 5 - 10g | 5-15g | 1 - 5 g |
| **06** | (NH₄)₂SO₄ | - | - | 0,2 - 4g |
| **07** | FeCl Solution ₃ 10% | - | - | 0,05 - 1mL |
| **08** | MnSO Solution ₄ 10% | - | - | 0,05 - 1mL |
| | Glycerol | - | - | 5 - 20mL |
| **10** | KNO3 | - | - | 0,5 - 3g |
| **09 11** | Malic acid | 1 - 5g | - | - |
| **12** | Sucrose | - | 5 -15g | - |
| **13** | Peptone | - | 2 - 5g | - |
| **14** | Water | q.s.p. IL | q.s.p. IL | q.s.p. IL |

| | | | | |
|---|---|---|---|---|
| q.s.p.: quantity sufficient for. | | | | |

**TABLE 3. CULTURE MEDIUM USED FOR GROWTH OF BACILLUS SPP. UP TO THE SCALE OF 10L.**

| | ***Reagents*** | *Bacillus* |
|---|---|---|
| **01** | K₂HPO₄ | 0,1 - 4 g |
| **02** | KH₂PO₄ | 0,1 - 4 g |
| **03** | MgSO_{4·}7H₂O | 0,1 - 0,6 g |
| **04** | NaCl | 0,05 - 0,3 g |
| **05** | Yeast extract | 0,1 - 4 g |
| **06** | Peptone | 0,2 - 4 g |
| **07** | FeCl Solution ₃ 10% | 0,05 - 1 mL |
| **08** | MnSO Solution ₄ 10% | 0,05 - 1 mL |
| **09** | Sucrose | 5 - 10 g |
| **10** | Water | q. s.p. 1L |

| | | |
|---|---|---|
| q.s.p.: quantity sufficient for. | | |

5

**TABLE 4. CULTURE MEDIUM USED FOR GROWTH OF B. AMYLOLIQUEFACIENS AND B. LICHENIFORMIS FOR THE SCALE UP TO 180 L AND 2.000 L CULTURE.**

| | ***Reagents*** | *B. amyloliquefaciens* | *B. licheniformis* |
|---|---|---|---|
| **01** | Yeast extract | 5-15 g | 1 - 5 g |
| **02** | Cornflour | - | 5-15 g |
| **03** | Sucrose | 5-15g | - |
| **04** | NaCl | 2-5 g | 2-5 g |
| **05** | Water | q.s.p. 1L | q.s.p. 1L |

| | | | |
|---|---|---|---|
| q.s.p.: quantity sufficient for. | | | |

Prior to the cultivation in the 2.000 L bioreactors there is carried out a process of sterilization of the culture medium for approximately 60 to 120 minutes, at a temperature of approximately 121 °C to approximately 130 °C. Preferably, the sterilization is carried out at a pressure of approximately 1,0 - 2,0 Kgf/cm². After the sterilization and cooling period, the tanks containing 180 L of the cultures of *Rhodopseudomonas, Lactobacillus, Bacillus amyloliquefaciensr B. licheniformis* and *Saccharomyces* cerevisiae are then inoculated in 2.000 L reactors containing the specific culture medium for each microorganism, previously described in tables 2, 3 and 4.

### EXAMPLE 2 - MIXTURE OF THE MICROORGANISMS IN BIOREACTOR OR MIXER

For obtaining the products with suitable homogeneity, the mixture of the species of *Rhodopseudomonas, Lactobacillus, Saccharomyces* and *Bacillus* occurs in bioreactors or 10.000 L mixer in a process of 0,5 to 2 hours under constant agitation of 40 hz to 45 hz.

### EXAMPLE 3 - ZOOTECHNICAL PERFORMANCE AND VIABILITY OF THE BIRDS TREATED WITH THE COMBINATION OF MICROORGANISMS PROPOSED BY THE PRESENT INVENTION.

For the characterization of the probiotic effect of the combination of microorganisms described in the present invention, Rhodopseudomonas palustris CCTB18, *Lactobacillus plantarum* CCTB27, *Bacillus amyloliquefaciens* CCTB06, *Bacillus licheniformis* CCTB07 and *Saccharomyces cerevisiae* CCTB12, there was carried out a trial containing three treatments;T1 -absolute control without addition of probiotic; T2 - positive control composed by commercial probiotic containing the mixture of *Bacillus subtilis* and *Bacillus licheniformis,* in powder form administered according to the manufacturer's recommendation (2 Kg of product per ton of feed); T3 - probiotic composition described in the present invention containing *Rhodopseudomonas palustris* CCTB18, Lactobacillus plantarum CCTB27, Bacillus amyloliquefaciens CCTB06, *Bacillus licheniformis* CCTB07 and *Saccharomyces cerevisiae* CCTB12 administered at the dose of 1 L for each 1.000 L water. Each treatment was composed of 10 replications with 30 birds each, totaling 300 animals per treatment. The animals used were male, with initial weight of 42 to 48 g and the study was carried out for 42 days.

During the trial there were evaluated, weekly, different zootechnical performance parameters such as the average weight per animal, average feed consumption, accumulated feed conversion, accumulated daily weight dain, productive efficiency index.

The temperature of the avian warehouse destined to the experiment had the temperature controlled according to the optimum level for each development stage of the animal. However, during the thirtieth and thirty-first days, the animals were submitted to a high temperature challenge for 48 hours at 32 °C. As from the thirty-second day, the temperature was once more stabilized between 26 °C and 28 °C, decharacterizing the heat stress (Table 5).

**TABLE 5: TEMPERATURE AT °C THROUGHOUT THE EXPERIMENT DAYS WITH THE TEMPERATURE CHALLENGE APPLIED ON THE THIRTIETH AND THIRTY-FIRST DAYS OF LIFE OF THE ANIMALS.**

| **Optimum temperature** | **Age in Days** | **Hour** | **°C** | **Hour** | **°C** | **Hour** | **°C** | **Hour** | **°C** | **°C Average** |
|---|---|---|---|---|---|---|---|---|---|---|
| **33 °C** | 1 | 07:30 | 33,00 | 11:00 | 33,10 | 14:00 | 33,40 | 18:00 | 33,40 | 33,23 |
| | 2 | 07:30 | 30,40 | 11:00 | 31,00 | 14:00 | 32,58 | 18:00 | 32,78 | 31,69 |
| | 3 | 07:30 | 32,18 | 11:00 | 32,20 | 14:00 | 33,20 | 18:00 | 31,84 | 32,36 |
| **28-31 °C** | 4 | 07:30 | 29,52 | 11:00 | 29,56 | 14:00 | 31,08 | 18:00 | 28,03 | 29,55 |
| | 5 | 07:30 | 28,03 | 11:00 | 28,05 | 14:00 | 32,16 | 18:00 | 29,22 | 29,37 |
| | 6 | 07:30 | 27,26 | 11:00 | 28,06 | 14:00 | 32,12 | 18:00 | 28,09 | 28,88 |
| | 7 | 07:30 | 27,08 | 11:00 | 29, 08 | 14:00 | 30,04 | 18:00 | 27,68 | 28,47 |
| **26-28 °C** | 8 | 07:30 | 27,08 | 11:00 | 27,09 | 14:00 | 28,40 | 18:00 | 28,09 | 27,67 |
| | 9 | 07:30 | 27,04 | 11:00 | 28,16 | 14:00 | 29,08 | 18:00 | 28,02 | 28,08 |
| | 10 | 07:30 | 26,05 | 11:00 | 27,02 | 14:00 | 27,01 | 18:00 | 27,56 | 26,91 |
| | 11 | 07:30 | 26,12 | 11:00 | 26,64 | 14:00 | 26,08 | 18:00 | 26,04 | 26,22 |
| | 12 | 07:30 | 26,62 | 11:00 | 26,26 | 14:00 | 28,06 | 18:00 | 27,04 | 27,00 |
| | 13 | 07:30 | 25,64 | 11:00 | 26,00 | 14:00 | 29,08 | 18:00 | 27,06 | 26,95 |
| | 14 | 07:30 | 26,28 | 11:00 | 26,98 | 14:00 | 30,02 | 18:00 | 29,08 | 28,09 |
| | 15 | 07:30 | 24,26 | 11:00 | 25,09 | 14:00 | 28,04 | 18:00 | 29,76 | 26,79 |
| | 16 | 07:30 | 24,30 | 11:00 | 26,02 | 14:00 | 27,82 | 18:00 | 28,25 | 26,60 |
| | 17 | 07:30 | 22,08 | 11:00 | 26,86 | 14:00 | 29,40 | 18:00 | 28,06 | 26,60 |
| | 18 | 07:30 | 23,00 | 11:00 | 27,02 | 14:00 | 28,08 | 18:00 | 26,16 | 26,07 |
| | 19 | 07:30 | 26,06 | 11:00 | 28,08 | 14:00 | 30,00 | 18:00 | 29,07 | 28,30 |
| | 20 | 07:30 | 24,08 | 11:00 | 27, 88 | 14:00 | 29,01 | 18:00 | 29,22 | 27,55 |
| | 21 | 07:30 | 24,98 | 11:00 | 27,05 | 14:00 | 30,06 | 18:00 | 27,09 | 27,30 |
| | **22** | 07:30 | 24,02 | 11:00 | 28,01 | 14:00 | 30,74 | 18:00 | 30,58 | 28,34 |
| **23-26 °C** | 23 | 07:30 | 25,62 | 11:00 | 27, 48 | 14:00 | 30,10 | 18:00 | 29,8 | 28,25 |
| | 24 | 07:30 | 26,64 | 11:00 | 30,74 | 14:00 | 29,94 | 18:00 | 30,24 | 29,39 |
| | 25 | 07:30 | 29,42 | 11:00 | 30,34 | 14:00 | 31,24 | 18:00 | 28,04 | 29,76 |
| | 26 | 07:30 | 26,16 | 11:00 | 26,02 | 14:00 | 26,09 | 18:00 | 26,02 | 26,07 |
| | 27 | 07:30 | 26,00 | 11:00 | 27,04 | 14:00 | 27,08 | 18:00 | 26,48 | 26,65 |
| | 28 | 07:30 | 26,16 | 11:00 | 27,64 | 14:00 | 29,36 | 18:00 | 30,46 | 28,41 |
| | 29 | 07:30 | 24,05 | 11:00 | 30,68 | 14:00 | 31,04 | 18:00 | 29,08 | 28,71 |
| | 30 | 07:30 | 31,38 | 11:00 | 32, 38 | 14:00 | 33,00 | 18:00 | 32,25 | 32,25 |
| | 31 | 07:30 | 31,02 | 11:00 | 31,4 | 14:00 | 33,21 | 18:00 | 33,14 | 32,19 |
| | 32 | 07:30 | 29,04 | 11:00 | 30,08 | 14:00 | 30,06 | 18:00 | 26,73 | 28,98 |
| | 33 | 07:30 | 28,18 | 11:00 | 30,03 | 14:00 | 31,04 | 18:00 | 26,02 | 28, 82 |
| | 34 | 07:30 | 25,48 | 11:00 | 26,06 | 14:00 | 28,08 | 18:00 | 28,94 | 27,14 |
| | 35 | 07:30 | 25,26 | 11:00 | 27,04 | 14:00 | 29,52 | 18:00 | 29,85 | 27,92 |
| | 36 | 07:30 | 26,26 | 11:00 | 27,11 | 14:00 | 27,02 | 18:00 | 27,88 | 27,07 |
| | 37 | 07:30 | 26,20 | 11:00 | 24, 14 | 14:00 | 25,78 | 18:00 | 26,92 | 25,76 |
| | 38 | 07:30 | 21,06 | 11:00 | 25,02 | 14:00 | 25,34 | 18:00 | 26,04 | 24, 37 |
| | 39 | 07:30 | 23,84 | 11:00 | 25, 41 | 14:00 | 25,41 | 18:00 | 25,5 | 25,04 |
| | 40 | 07:30 | 24,16 | 11:00 | 24,9 | 14:00 | 26,54 | 18:00 | 26,04 | 25,41 |
| | 41 | 07:30 | 26, 10 | 11:00 | 25,04 | 14:00 | 26,84 | 18:00 | 25,11 | 25, 77 |
| | 42 | 07:30 | 24,14 | 11:00 | 28, 14 | 14:00 | 29,10 | 18:00 | 28,51 | 27,47 |

Applying the high temperature challenge (heat stress) for 48 hours during the thirtieth and thirty-first days of life of the animals caused a behavior change in different zootechnical parameters evaluated.

Surprisingly, except for the average weight of the animals (PV), all the other parameters, average feed consumption (GPD), accumulated feed conversion (FC), accumulated daily weight gain (FCR) and productive efficiency index (IEP), were significantly altered at 42 days of life of the animals by the treatment corresponding to the probiotic additive, object of the present invention (T3) (Tables 7, 8, 9, 10 and 11).

**TABLE 7: RESULTS OBTAINED FOR AVERAGE WEIGHT OF 10 PER TREATMENT. ASSESSMENTS CARRIED OUT ON THE 7, 14, 21, 28, 35 AND 42 DAYS OF LIFE OF THE ANIMALS.**

| **TREATMEN T** | **PV 7d *ns** | **PV 14d *ns** | **PV 21d *ns** | **PV 28d *ns** | **PV 35d *ns** | **PV 42d *ns** |
|---|---|---|---|---|---|---|
| T1 | 182,00 | 495,20 | 1013,74 | 1702,78 | 2367,53 | 3286,5 |
| T2 | 181,00 | 496, 98 | 1020,76 | 1704,49 | 2362,85 | 3220,2 |
| T3 | 181, 00 | 502,38 | 1008, 29 | 1673,98 | 2382,11 | 3175,8 |

Averages of 10 birds per replication (10 replications). *ns: no statistical difference between the treatments by test-t(p≤0,05).

**TABLE 8: RESULTS OBTAINED FOR AVERAGE FEED CONSUMPTION PER TREATMENT. ASSESSMENTS CARRIED OUT ON THE 7, 14, 21, 28, 35 AND 42 DAYS OF LIFE OF THE ANIMALS.**

| **TREATMENT** | **GPD 7d *ns** | **GPD 14d *ns** | **GPD 21d *ns** | **GPD 28d *ns** | **GPD 35d *ns** | **GPD 42d** |
|---|---|---|---|---|---|---|
| T1 | 19,80 | 32,26 | 46,20 | 59,26 | 66,4001 | 77,214 a |
| T2 | 19,70 | 32,39 | 46, 53 | 59,32 | 66,2653 | 75,6317 b |
| T3 | 19,70 | 32,77 | 45,94 | 58,23 | 66,8165 | 74,5782 b |

Averages (10 replications) followed by the same letter in the same column do not present statistical difference between the treatments by the test-t (p≤0,05). *ns: no statistical difference between the treatments.

**TABLE 9: RESULTS OBTAINED FOR THE ACCUMULATED FEED CONVERSION BY TREATMENT. ASSESSMENTS CARRIED OUT ON THE 7, 14, 21, 28, 35 AND 42 DAYS OF LIFE OF THE ANIMALS.**

| **TREATMENT** | **FC 7d *ns** | **FC 14d *ns** | **FC 21d *ns** | **FC 28d *ns** | **FC 35d** | **FC 42d** |
|---|---|---|---|---|---|---|
| T1 | 153,00 | 514,61 | 1187,43 | 2304,55 | 3394,8 a | 4677,03 a |
| T2 | 154,00 | 536,77 | 1208,41 | 2330,84 | 3439,7 a | 4797,98 a |
| T3 | 157,00 | 535,50 | 1211,16 | 2336,18 | 3548,2 b | 5081,85 b |

Averages (10 replications) followed by the same letter in the same column do not present statistical difference between the treatments by the test-t (p≤0,05). *ns: no statistical difference between the treatments.

**TABLE 10: RESULTS OBTAINED FOR ACCUMULATED DAILY WEIGHT GAIN PER TREATMENT. ASSESSMENTS CARRIED OUT ON THE 7, 14, 21, 28, 35 AND 42 DAYS OF LIFE OF THE ANIMALS.**

| **TREATMENT** | **FCR 7d *ns** | **FCR 14d *ns** | **FCR 21d *ns** | **FCR 28d *ns** | **FCR 35d** | **FCR 42d** |
|---|---|---|---|---|---|---|
| T1 | 0,842 | 1,049 | 1,169 | 1,417 | 2,4889 a | 2,05638 a |
| T2 | 0,849 | 1,091 | 1,203 | 1,419 | 2,3117 a | 2,07075 a |
| T3 | 0,850 | 1,080 | 1,212 | 1,435 | 1,736 b | 1,8421 b |

Averages (10 replications) followed by the same letter in the same column do not present statistical difference between the treatments by the test-t (p≤0,05). *ns: no statistical difference between the treatments.

**TABLE 11: RESULTS OBTAINED FOR THE CUMULATIVE PRODUCTIVE EFFICIENCY INDEX BY TREATMENT. ASSESSMENTS CARRIED OUT ON THE 7, 14, 21, 28, 35 AND 42 DAYS OF LIFE OF THE ANIMALS.**

| **TREATMENT** | **IEP 7d** | **IEP 14d** | **IEP 21d** | **IEP 28d** | **IEP 35d** | **IEP 42d** |
|---|---|---|---|---|---|---|
| T1 | 235,000 | 300,95 | 390,48 | 395,00 | 183,469 a | 222,804 a |
| T2 | 231,000 | 293,31 | 378,89 | 397,56 | 191,59 a | 219,076 a |
| T3 | 232,000 | 300,23 | 376,57 | 392,43 | 323,852 b | 336,124 b |

Averages (10 replications) followed by the same letter in the same column do not present statistical difference between the treatments by the test-t (p≤0,05). *ns: no statistical difference between the treatments.

The results presented for the zootechnical parameters evaluated demonstrated the effectiveness of the microbiological combination proposed by the present invention (treatment 3), through which the preventive role of the use thereof became evident from the initial steps of the lifecycle of the animals evaluated. Additionally, by means of the analysis of the results for the different zootechnical parameters, it is possible to evidence that the combination of microorganisms proposed by the technical solution presents superior results when compared with the commercial probiotic product, which uses species recognized by the state of the art, herein represented by treatment 2.

Still for the same trial, it was assessed the viability of the birds throughout the 42 days of the experiment. Surprisingly, for this parameters, treatment 3 (probiotic composition object of the present invention) presented superior results to treatments 1 (absolute control) and treatment 2 (commercial probiotic) (table 12).

**TABLE 12: RESULTS EXPRESSED IN PERCENTAGE FOR THE VIABILITY OF THE ANIMALS IN DIFFERENT TREATMENTS. ASSESSMENTS CARRIED OUT ON THE 7, 14, 21, 28, 35 AND 42 DAYS OF LIFE OF THE ANIMALS.**

| TREATMENT | (%) 7d *ns | (%) 14d *ns | (%) 21d *ns | (%) 28d *ns | (%) 35d | (%) 42d |
|---|---|---|---|---|---|---|
| T1 | 100,00 | 100,00 | 100,00 | 93,33 | 51,665 a | 51,665 a |
| T2 | 100,00 | 100,00 | 98,34 | 93,33 | 63,335 a | 63,335 a |
| T3 | 100,00 | 100,00 | 100,00 | 96,67 | 85 b | 83,33 b |

Values followed by the same letter in the same column do not present statistical difference between the treatments by the Mann-Whitney test (p≤0,05). *ns: no statistical difference between the treatments.

The preventive effect of the microbiological combination proposed, *R. palustris, L. plantarum, B. amyloliquefaciens, B. licheniformisand Saccharomyces cerevisiae,* was effective in the prevention of the deleterious effects caused by heat stress, now represented by the mortality rate of the birds submitted to the imposed challenge, temperature stress (32 °C) for 48 hours, between the thirtieth and thirty-first days of life of the animals.

## Claims

1. **PROBIOTIC COMPOSITION FOR HIGHER HEAT STRESS TOLERANCE IN POULTRY, characterized by** comprising the microbiological combination of: 50.001% to 99.995% of *Rhodopseudomonas palustris,* 0.001% to 49.995% of *Lactobacillus plantariam,* 0.001% to 49.995% of *Bacillus amyloliquefaciens,* 0.001% to 49.995% of *Bacillus licheniformis,* as well as 0.001% to 49.995% of a species of yeast-like fungus, in aqueous carrier, directed to treating poultry at high temperature.

2. **COMPOSITION,** according to claim 1, **characterized by** the preferred strains being *Rhodopseudomonas palustris* CCTB18, *Lactobacillus plantarum* CCTB27, *Bacillus amyllllquefaclens* CCTB06, *Bacillus licheniformis* CCTB07 and that the yeast-like fungus will preferably have 0.001% to 49.995% *Saccharomyces cerevisiae* CCTB12.

3. **COMPOSITION,** according to claims 1 and 2, **characterized by** being able to additionally have excipients, veterinary carriers and other products suitable to oral treatment of poultry with heat stress by feed supplements.

4. **METHOD OF PRODUCING THE PROBIOTIC COMPOSITION FOR HIGHER TOLERANCE TO HEAT STRESS IN POULTRY, characterized by** comprising the following steps:
A) The different species are cultivated separately by batch fermentation processes, **characterized by** applying different culture medium compositions for each microorganism used in the present invention;
B) The scaling of the batch fermentation processes uses different steps, namely: cultivation in 100 mL, cultivation of 1 L, cultivation of 10 L, cultivation of 180 L and, finally cultivation of 2.000 L, not being restricted only to these volumes;
C) the species of *Rhodopseudomonas, Lactobacillus, Bacillus* and *Saccharomyces* are expanded by incubation under temperature of approximately 22 °C to 38 °C;
D) the species of *Rhodopseudomonas, Lactobacillus, Bacillus* and *Saccharomyces* are expanded, 100 mL step, by incubation in orbital agitator at 80 rpm to 200 rpm;
E) the species of *Rhodopseudomonas, Lactobacillus, Bacillus* and *Saccharomyces* are expanded by incubation for 8 hours to 168 hours, depending on the different steps of 100 mL, 1 L, 10 L, 180 L and 2.000 L;
F) the species of *Rhodopseudomonas, Lactobacillus* and *Saccharomyces* are expanded in glass flasks, containing 1 L of specific culture medium, and *Bacillus* are expanded in stainless steel balloons containing 1 L of specific culture medium, and 10 L culture medium in stainless-steel balloons;
G) the species of *Rhodopseudomonas, Lactobacillus, Bacillus* and *Saccharomyces* are incubated with airflow at 0,25 Nm³/h to 10,0 Nm³/h, depending on the different steps of 1 L, 10 L, 180 L and 2.000 L; and
H) the fermentation step of 2.000 L is conducted with agitation of 40 hz to 45 hz, finally obtaining the liquid probiotic composition, as defined in claims 1 to 3, directed to administering to poultry with heat stress.

5. **METHOD,** according to claim 4, **characterized by** the fact that the administration is, preferably, through the drinking water.

6. **METHOD,** according to claims 4 and 5, **characterized by** the fact that the administration must be carried out, preferably from the first days of life of the animals.

7. **METHOD,** according to any of claims 4 to 6, **characterized by** the fact that the effect is preventive.

8. **METHOD,** according to any of claims 4 to 7, **characterized by** increasing the performance in poultry submitted to heat stress according to the zootechnical parameters of average feed consumption, accumulated feed conversion, accumulated daily weight gain, accumulated productive efficiency index.

9. **METHOD,** according to any of claims 4 to 8, **characterized by** increasing the viability of poultry submitted to heat stress.

10. **USE OF A PROBIOTIC COMPOSITION characterized by** said composition being defined in claims 1 to 3 and produced by method defined in claims 4 to 9, and being for manufacturing a veterinary feed supplement, directed to increasing the viability of poultry submitted to heat stress.

11. **USE,** according to claim 10, **characterized by** the veterinary feed supplement being administered, preferably, from the first stages of life of the animals, being capable of being preventive, and being preferably administered through the drinking water, increasing the performance of birds submitted to heat stress according to the zootechnical parameters for average feed consumption, accumulated feed conversion, accumulated daily weight gain, accumulated productive efficiency index.
